# EUROPÄISCHE PATENTANMELDUNG

(11) **EP 2 380 511 A2**
(43) Veröffentlichungstag der Anmeldung: **26.10.2011**
(21) Anmeldenummer: 11163421.8
(22) Anmeldetag: 21.04.2011
(51) Int. Cl.: A61B 17/32

(54) **Chirurgisches Instrument, insbesondere Ultraschallschere**

(30) Priorität: 22.04.2010 DE 202010005727 U
(71) Anmelder: Söring GmbH, 25451 Quickborn (DE)
(72) Erfinder: Jozat, Walter, 24576 Bad Bramstedt (DE); Meier, Markus, 25337 Elmstrom (DE); Jaudszims, Andreas, 24558 Henstedt-Ulzburg (DE)
(74) Vertreter: Glawe, Delfs, Moll

(57) **Zusammenfassung**

Die vorliegende Erfindung betrifft chirurgisches Ultraschall-Klemm- und/oder Schneidinstrument bestehend aus einem Gehäuse (5') mit angeformtem Handgriff (5), einer in das Gehäuse (5') eingesteckten Konvertereinheit (2) mit hierin befestigter Sonotrode (16), einem im Gehäuse (5') gelagerten Betätigungsgriff (6), einem im Gehäuse (5') gelagerten und aus diesem vorstehenden Schubrohr (10) zur Betätigung der Klemme (18) und einem Führungsrohr (23) zur Aufnahme der Sonotrode (16), wobei die im Gehäuse angeordnete Einheit mittels eines Sternrades (12) um die Längsachse gedreht werden kann, dadurch kennzeichnet, dass das Führungsrohr (23) in dem Schubrohr (10) gelagert, wobei das Schubrohr (10) begrenzt in Längsrichtung verschiebbar ist, um die Klemme (18) zu öffnen und/oder zu schließen und um bei diesem Vorgang eine Wegdifferenz zwischen Klemme (18) und Sonotrode (16) zu erreichen.

## Beschreibung

Die vorliegende Erfindung bezieht sich auf chirurgische UltraschallInstrumente, Insbesondere auf chirurgische Ultraschall-Klemmund/oder Schneldinstrumente mit einer separat drehbaren Sonotrode und darüber hinaus mit einer Möglichkeit der Drehung des Gesamtgerätes gegenüber dem Handgriff der Schere.

Chirurgische Ultraschallinstrumente finden aufgrund Ihrer vielseitigen Einsatzmöglichkeiten immer breitere Einsatzgebiete, Je nach konstruktiver Gestaltung Können chirurgische Ultraschallinstrument sowohl zum Schneiden als auch zum Koagulieren eingesetzt werden. Je nach Anforderung wird entweder eine Sonotrode mit Schneidkante oder eine Sonotrode mit stumpfer Kante verwendet.
Aus dem Stand der Technik -DE 698 14 282 T2- ist eine Lösung bekannt, bei der mit einer ersten Drehmöglichkeit die mit zwei unterschiedlichen Längskanten ausgestattete Sonotrode relativ zur Klemmbacke gedreht werden kann. Außerdem wird eine zweite Drehmöglichkeit beschrieben, bei der die gesamt Einheit innerhalb des Griffstücks gedreht werden kann. Dieses soll den Vorteil haben, dass der Chirurg während eines operativen Eingriffs nicht mehr das ganze Instrument -also Handgriff, Konverter und Schereneinheitdrehen muss, sondern dass er den Handgriff In der ihm bequemen Lage belassen kann und nur den Konverter mit Schereneinheit in dem Handgriff zu drehen braucht.
Das wird dadurch bewerkstelligt, dass Sonotrode, Klemm-Mechanismus und Ulttaschall-Antriebselnheit als Einheit gedreht worden, wobei ein Rastmechanismus dazu dient, eine indexierte Drehposition von Klemm-Mechanismus und Sonotrode relativ zum Gehäuse zu ermöglichen.

In der US-PS 6024750 wird ein Ultraschall-Instrument beschrieben, welches zum Schneiden und/oder Koagulieren eingesetzt werden soll. Dieses Instrument enthält einen drehbaren Kragen, welcher mit dem Konverter, der Klemmeinrichtung und dem Scherenmaul derart zusammen wirkt, dass eine Drehung des drehbaren Kragens eine Drehung von Konverter, Klemmeinrichtung und Scherenmaul um die Längsachse des Gerätes bewirkt.

Bei all diesen vorbekannten Geräten handelt es sich um sogenannte Elnweg-Geräte, d.h. nach jedem operativen Einsatz muss das Gerät entsorgt werden. Eine solche Lösung ist aus Kostengründen abzulehnen.

Der Erfindung liegt somit die Aufgabe zugrunde, ein In einem GriffStück drehbar angeordnetes chirurgisches Ultraschall-instrument zu schaffen, welches aus einem Konverter, einer Sonotrode mit zugehöriger Klemm- und/oder Schneideinrichtung und einem Griffstück besteht und welches mit wenigen Handgriffen zerlegbar ist, damit es im Autoklaven keimfrei gemacht werden kann. Zu Aufgabe gehört auch, dass die Einzelteile der Schere nach dem Autoklavieren problemlos und schnell zusammen gebaut werden können.

Gelöst wird diese Aufgabe dadurch, dass die einzelnen Bauteile 80 gestaltet sind, dass sie nur in einer einzigen Position -der Funktionsposition- montiert werden können.

Die Funktionseinheit -Konverter, Sonotrode und Klemmbacke- ist so gestaltet, dass sie vorzugsweise bei nicht betätigten Betätigungshebein beschränkt, d.h. um einen bestimmten Winkel oder auch komplett um die eigene Achse drehbar ist. Hierbel sind selbstverständlich auch unterschiedliche Formen der Klemmbacke und der entsprechenden Sonotrode denkbar. Diese konstruktive Lösung hat für den Chirurgen den Vorteil, dass er das Griffstück in der für ihn bequemsten Haltung in der Hand halten kann und der Konverter mit Sonotrode und Klemmbacke In die für die Durchführung der Operation günstigste Position gedreht werden kann. Um ein unbeabsichtigtes Verdrohen zu vermeiden, kann die Einheit innerhalb des Handstückes mittels einer Klemmschraube oder eines Brems- oder Klemmhebels festgesetzt werden. Denkbar ist auch, dass nach Betätigung der Betätigungshebel automatisch eine die Rotation behindernde Bremse eingreift, damit während der Durchführung einer Operation ein unbeabsichtigtes Verdrehen vermieden werden kann.

Weitere Gestaltungsmerkmale der Erfindung sind In den Unteransprüchen beschrieben.

Die allgemeine Kostenentwicklung im Gesundheitswesen macht es erforderlich, dass auch im Operationsbereich die Einmalgeräte soweit möglich- durch mehrfach verwendbare Geräte ersetzt werden. Für den Einsatz von Ultraschallscheren bedeutet das, dass diese so gestaltet werden, dass nach dem Entfernen von Zuleitung, Konverter und Sonotrode mit Klemmbacke die Baueinheit "Schere" in einem Autoklaven sterilisiert werden kann. Hierzu ist es erforderlich, dass das Instrument leicht, d.h. ohne zusätzliches Werkzeug, zerlegt und nach der Autoklavierung ebenso leicht wieder zusammengebaut werden kann. Dies wird durch den erfindungsgsmäßen Aufbau erreicht.

Anhand eines Ausführungsbeispieles soll der Erfindungsgegenstand näher beschrieben werden. Es zeigt:
- Fig. 1: eine Seitenansicht eines Ultraschall-Instrumentes mit Konverter, Betätigung und Schere,
- Fig. 2: einen tellweisen Schnitt durch das Instrument aus Figur 1
- Fig.: 3 einen Ausschnitt Z aus Figur 2 In vergrößerter Darstellung,
- Fig. 4: einen Schnitt gem. der Linie B-B aus Fig. 2 In vergrößerter Darstellung.

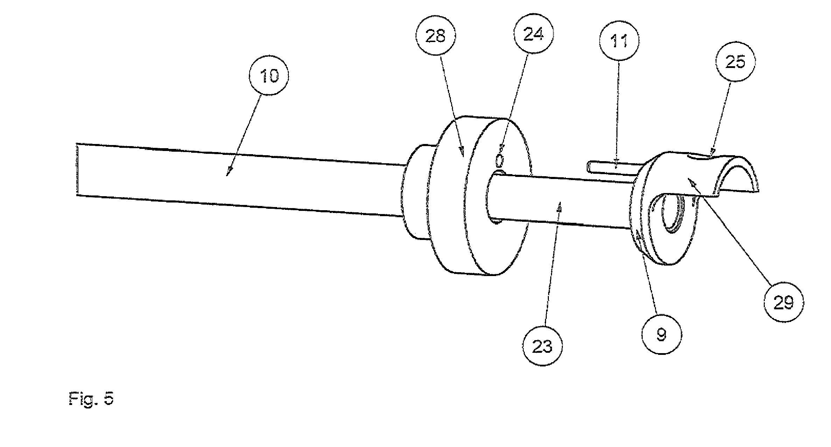

- Fig. 5: eine perspektivische Darstellung von SonotrodenSchutzrohr und Schubrohr.

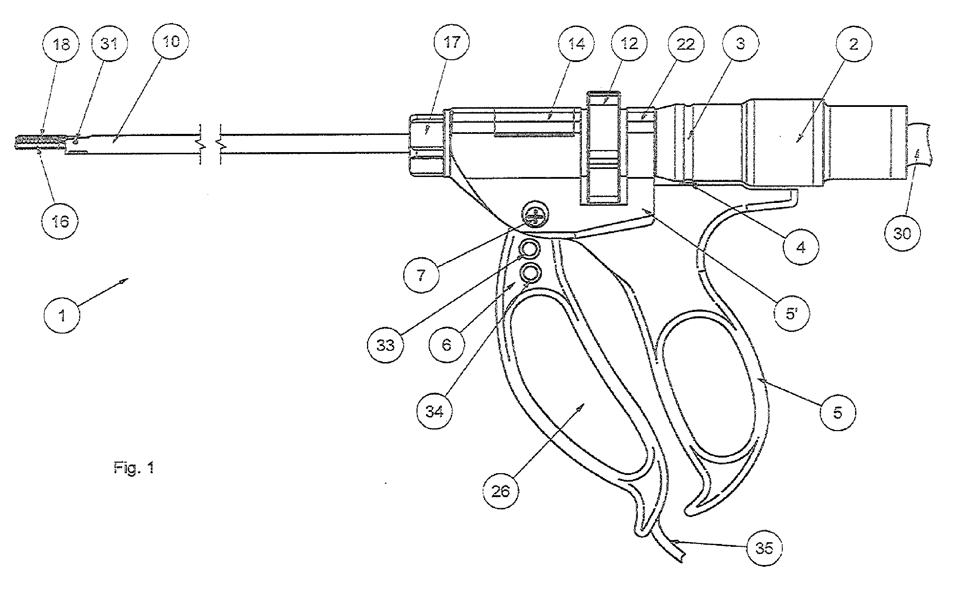

Figur 1 zeigt eine Ultraschallschere (1) in der Seitenansicht. Diese Schere (1) besteht aus einem Gehäuse (5') mit angeformtem Handgriff (5) und einer Konverteraufnahme (22), in der der Konverter (2) befestigt ist. Zur Sicherstellung, dass der Konverter (2) richtig in das Gehäuse (6') eingesetzt ist, ist eine Fixiernut (3) vorgesehen, In die ein Rastnocken (4) eingreift. Mittels des Drehgriffs (12) kann die gesamte Einheit, d.h. Konverter (2), Schubrohr (10), Sonotrode (16) und Klemme (18) um die Längsachse gedreht werden. Die Drehbarkeit dieser Einheit kann z.B. mittels in der Fixiernut (3) vorgesehenen Vertiefungen (hier nicht dargestellt) schrittweise erfolgen. Es ist auch denkbar, das am Gehäuse eine Foststelleinrichtung vorgesehen ist, mittel der die Einheit nach dem Einstellen der bevorzugten Position festgestellt worden kann.
Der Betätigungsgriff (6) mit der Grifföffnung (26) ist durch Lösen und Entfernen der Schraube (7) leicht gegen einen anders geformten - z.B. einen geschlossenen- Griff austauschbar. Die Lagerung des Schubrohres ist mittels eines aufgesteckten Deckels (14) abgedeckt. Denkbar ist auch ein Griff (6), in den ein oder mehrere Schalttaster (33,34) für unterschiedliche Funktionen z.B. "Schneiden" oder "Koagulieren" integriert sind. Diese ermöglichen es dem Chirurgen, die Ultraschallschere sowohl über die handbetätigten Schalttaster (33,34) als auch über einen -hier nicht dargestellten- Fußschalter zu aktivieren. Am Griff können die erforderlichen Kabel (35) beispielsweise als Steckverbindung vorgesehen sein.

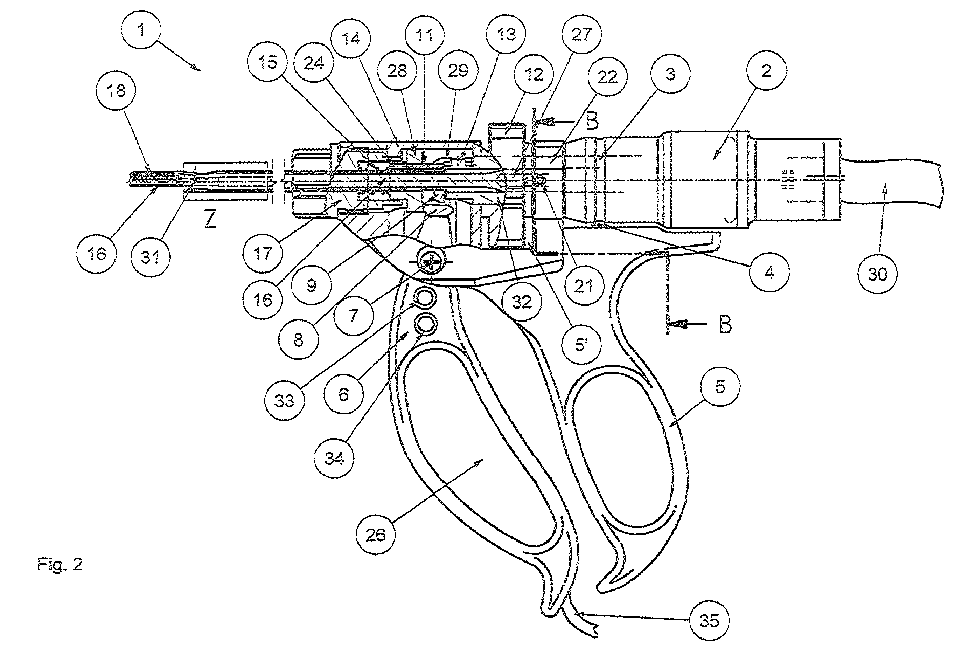

Figur 2 zeigt die vorbeschriebene Ultraschallschere (1) in einer teilweise geschnittenen Ausführung. Hier ist klar zu erkennen, dass das Führungsrohr (23) mit einem Kragen (9) versehen ist, an den die halbringförmige Lagerschale (29) angeformt ist. Achsparallel ist in diesen Kragen ein Führungsstift (11) eingebracht, der in die Aufnahmebohrung (24) im Schubring (28) ragt und hierin gleitet. In der Lagerschale (29) ist quer zur Langsachse eine Arretierbohrung (25) vorgesehen, in welche der Aufnahmezapfen (13) hineinragt. Durch diese Konstruktion ist sicher gestellt, dass Schubrohr (10) und Führungsrohr (23) in der konstruktiv vorbestimmten Position zu einander verbunden sind, damit die Klemmfläche der Klemme (18) korrekt auf der Sonotrode aufliegt. Die Sonotrode wird innerhalb des Führungsrohres (23) durch hier nicht dargestellte Stützringe -z.B. Silikon- oder Gummiringe- geführt. Mittels einer Fixierschraube (21) wird sicher gestellt, dass die Sonotrode (16) nur in einer vorbestimmten Position in das Gehäuse hinein geschoben werden kann. Durch Verlagerung des Betatigungsgriffs (6), der un die Achse der Schraube (7) schwenkbar ist, der am Betätigungsgriff (6) angeformte Schubkopf (8) gegen den Schubring (28). Hierdurch wird das am Schubring (28) befestigte Schubrohr (10) in Richtung Klemme (18) gegen die Kraft der Rückstellfeder (15) verlagert Durch diese Bewegung wird die Klemme (18) verschwenkt und mit ihrer Klemmfläche gegen die Sonotrode (16) gedrückt. Nach dem Loslassen des Betätigungsgriffes (6) wird das Scherenmaul geöffnet und die Klemme (18) somit von der Sonotrode weggeschwenkt. Im Bereich der Lagerung (19) der Klemme (10), die um die Achse (31) geschwenkt wird, ist Im Führungsrohr (23) ein Lager (20) für den Endbereich der Sonotrode (16) vorgesehen.
Entgegen der bisher bekannten Ultraschalischeren vollzieht bei diesem Bewegungsablauf die Klemme (18) einen zur Sonotrode (16) vorgegebenen Weg, der sich aus dem Öffnungs- bzw. Schließwinkel der Klemme (18) ergibt.
Dies hat den Vorteil, dass das zu behandelnde Körpergewebe beim Schließen der Klemme weiter nach vorne in den maximalen Ultraschall-Wirkbereich der Sonotrode (16) geschoben wird.
Soll nun zu Reinigungszwecken die Schere (1) zerlegt werden, werden zunächst -nachdem Kabel und Schläuche entfernt wurden- die Einschraubführung (17) herausgeschraubt und die Abdeckung (14) entfernt dann der Konverter (2) mit der daran befestigten Sonotrode (16) aus dem Griffteil herausgezogen. Anschließend wird die Lagerschale (29) durch leichtes "Abwinkeln" von dem an der Welle (32) angeformten Aufnahmezapfen (13) abgehoben und zusammen mit dem Schubrohr (10) und dem Führungsrohr (23) aus dem Grifftell herausgezogen. Jetzt können alle Teile die keimfrei gemacht werden müssen, In einem Autoklaven sterilisiert werden.

Die Montage der Ultraschatischere (1) erfolgt dann in umgekehrter Reihenfolge wie die Demontage.

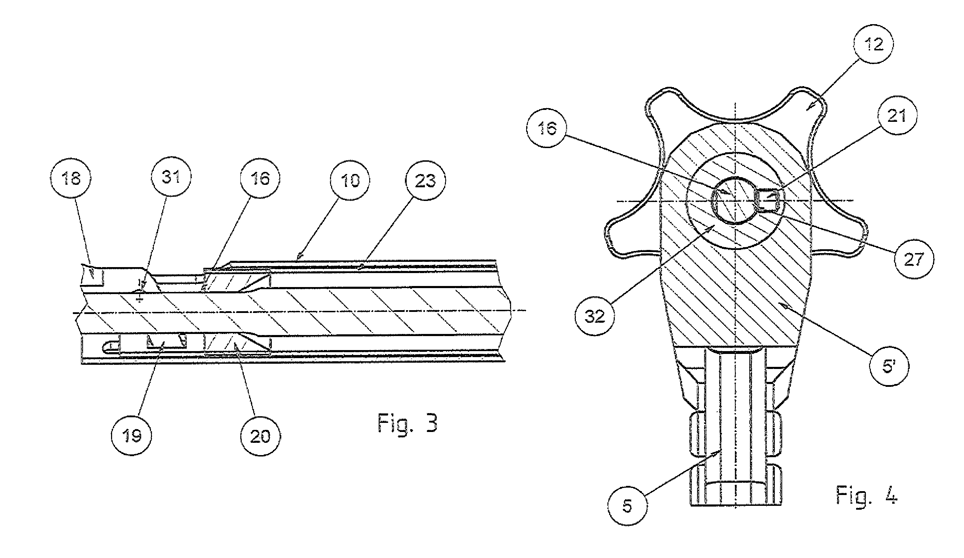

Figur 3 zeigt in vergrößerter Darstellung den Bereich "Z" aus Figur 2. Hierin ist zu sehen, dass sich im Führungsrohr (23) ein Lager (20) für die Sonotrode (16) befindet. Außerdem ist zu erkennen, dass die Klemme (18) mit dem Klemmenlager (19) durch eine Durchbrechung im Führungsrohr (23) hindurchragt und im Schubrohr (10) eingesetzt ist. Um die Schwenkachse (31), die im Führungsrohr (23) befestigt ist, wir die Klemme (16) in die Offenposition bzw. die Geschlossen-Position verschwenkt.

Figur 4 zeigt eine Schnittdarstellung entspr. der Kennzeichnung B - B aus Figur 2. Aus diesen beiden Figuren wird deutlich, dass im Gehäuse 5' eine Welle (32) gelagert ist, auf die der Konverter (2) aufgesteckt ist, Bevor der Konverter (2) aufgesteckt wird, wird die Sonotrode (16) in eine -hier nicht dargestellte- Bohrung eingeschraubt. Der Konverter (2) wird soweit auf die Welle aufgeschoben bis der Rastnocken (4) in die Fixiernut (3) einrastet. Um die eindeutige Position der Sonotrode (16) zu gewährleisten, wird eine Fixierschraube (21) eingeschraubt, deren Ausrichtfläche (27) auf die an der Sonotrode (16) angebrachte Fläche drückt. Hierdurch wird sichergestellt, dass die Sonotrode (16) beim Zusammenbau der Ultraschallschere (1) nur in der vorbestimmten Position eingeschoben werden kann.

Auf die Welle (32) ist das Sternrad (12) aufgeschoben und mit einer --hier nicht dargestellten- Klemmschraube befestigt. Es sind auch andere Arten der sicheren Verbindung -z.B. Aufschrumpfen, kleben etc- denkbar.

### Positionsnummernliste:

- 1 -: Ultraschallschere
- 2 -: Konverter
- 3 -: Fixiernut
- 4 -: Rastnocken
- 5 -: Handgriff
- 5' -: Gehäuse
- 6 -: Betätigungsgriff
- 7 -: Schraube
- 8 -: Schubkopf
- 9 -: Kragen
- 10 -: Schubrohr
- 11 -: Führungsstift
- 12 -: Sternrad
- 13 -: Aufnahmezapfen
- 14 -: Abdeckung
- 15 -: Feder
- 16 -: Sonotrode
- 17 -: Einschraubführung
- 18 -: Klemme
- 19 -: Klemmenlager
- 20 -: Lager
- 21 -: Fixierschraube
- 22 -: Konverteraufnahme
- 23 -: Führungsrohr
- 24 -: Aufnahmebohrung
- 25 -: Arretierbohrung
- 26 -: Grifföffnung
- 27 -: Ausrichtfläche
- 28 -: Schubring
- 29 -: Lagerschale
- 30 -: Verbindungsleitung
- 31 -: Schwenkachse
- 32 -: Welle
- 33 -: Schalttaster
- 34 -: Schalttaster
- 35 -: Anschlusskabel

## Patentansprüche

1. Chirurgisches Ultraschall-Klemm- und/oder Schneidinstrument bestehend aus:
einem Gehäuse (5') mit angeformiem Handgriff (5),
einer In das Gehäuse (5') eingesteckten Konvertereinheit (2) mit hierin befestigter Sonotrode (16),
einem im Gehäuse (5') gelagerten Betätigungsgriff (6),
einem im Gehäuse (5') gelagerten und aus diesem vorstehenden Schubrohr (10) zur Betätigung der Klemme (18) und einem Führungsrohr (23) zur Aufnahme der Sonotrode (16), wobei die Im Gehäuse angeordnete Einheit mittels eines Sternrades (12) um die Längsachse gedreht werden kann,
**dadurch gekennzeichnet, dass** das Führungsrohr (23) In dem Schubrohr (10) gelagert ist und dass das Schubrohr (10) begrenzt In Längsrichtung verschiebbar ist, um die Klemme (18) zu öffnen und/oder zu schließen und um bei diesem Vorgang eine Wegdifferenz zwischen Klemme (18) und Sonotrode (16) zu erreichen.

2. Chirurgisches Ultraschall-Klemm- und/oder Schneidinstrument nach Anspruch 1, **dadurch gekennzeichnet, dass** das Führungsrohr (23) mit einem Kragen (9) und einer mit diesem Kragen verbundenen Lagerschale (29) versehen ist zur formschlüssigen Verbindung mit der Konverteraufnahmewelle (32).

3. Chirurgisches Ultraschall-Klemm- und/oder Schneidinstrument nach Anspruch 2, **dadurch gekennzeichnet, dass** an der Konverteraufnahmewelle (32) ein radial vorstehender Aufnahmezapfen (13) vorgesehen ist, der in die Arretierbohrung (26) der Lagerschale (29) eingreift.

4. Chirurgisches Ultraschall-Klemm- und/oder Schneidinstrument nach Anspruch 1, **dadurch gekennzeichnet, dass** das Sternrad (12) form- oder kraftschlüssig mit der Konverteraufnahmewelle (32) verbunden ist.

5. Chirurgisches Ultraschall-Klemm- und/oder Schneidinstrument nach Anspruch 2, **dadurch gekennzeichnet, dass** in den Kragen (9) der Lagerschale (29) achsparallel ein Führungsstift (11) eingesetzt ist, der gleitend in die Aufnahmebohrung (24) das Schubringes (28) mündet und somit ein Verdrehen das Schubrohres gegenüber dem Führungsrohr verhindert,

6. Chirurgisches Ultraschall-Klemm- und/oder Schneidinstrument nach einem oder mehreren der vorhergehenden Ansprüche, **dadurch gekennzeichnet, dass** der mit dem Konverter (2) direkt verbundene Endbereich der Sonotrode mit einer Ausrichtfläche (27) versehen ist, die eine Montage in nur einer bestimmten Position gestattet.

7. Chirurgisches Ultraschall-Klemm- und/oder Schneidinstrument nach Anspruch 6, **dadurch gekennzeichnet, dass** der dem Konverter (2) zugewandte Endbereich der Sonotrode (16) einen unregeimäßigen polygonalen Querschnitt aufweist, der mit der Aufnahmeöffnung der Konverteraufnahmewelle (32) korrespondiert und eine Montage in nur einer Position zulässt,

8. Chirurgisches Ultraschall-Klemm- und/oder Schneidinstrument nach einem oder mehreren der vorhergehenden Ansprüche, **dadurch gekennzeichnet, dass** zur Abstützung der Sonotrode (16) im Führungsrohr (23) In regelmä-βigen Abständen Stützringe vorgesehen sind.

9. Chirurgisches Ultraschall-Klemm- und/oder Schneidinstrument nach einem oder mehreren der vorhergehenden Ansprüche, **dadurch gekennzeichnet, dass** im Gehäuse (5') ein Rastnocken (4) vorgesehen ist, der in die Nut (3) des Konverters (2) eingreift.

10. Chirurgisches Ultraschall-Klemm- und/oder Schneidinstrument nach Anspruch 8, **dadurch gekennzeichnet, dass** im Grund der Nut (3) In bestimmten Abständen zu einander Rastmulden vorgesehen sind.

11. Chirurgisches Ultraschall-Klemm- und/oder Scheidinstrument nach einem oder mehreren der vorhergehenden Ansprüche, **dadurch gekennzeichnet, dass** auf die Klemme (18) eine auswechselbare Druckplatte aufgesetzt ist.

12. Chirurgisches Ultraschall-Klemm- und/oder Schneidinstrument nach Anspruch 11, **dadurch gekennzeichnet, dass** die Druckplatte aus Kunststoff, vorzugsweise aus PTFE besteht.

13. Chirurgisches Ultraschall-Klemm- und/oder Schneidinstrument nach einem oder mehreren der vorhergehenden Ansprüche, **dadurch gekennzeichnet, dass** der Betätigungsgriff (6) im Kontaktbereich mit dem Schubring (28) mit einem Schubkopf aus PTFE ausgestattet ist.

14. Chirurgisches Ultraschall-Klemm- und/oder Schneidinstrument nach einem oder mehreren der vorhergehenden Ansprüche, **dadurch gekennzeichnet, dass** der Betätigungsgriff (6) gegen einen Griff in anderer Form auswechselbar ist, wobei der Betätigungsgriff (6) vorzugsweise besonders für Rechtshänder oder besonders für Linkshänder gestaltet ist.

15. Chirurgisches Ultraschall-Klemm- und/oder Schneidinstrument nach einem oder mehreren der vorhergehenden Ansprüche, **dadurch gekennzeichnet, dass** der Betätigungsgriff (6) gegen einen Griff mit integrierten Schalttastern (33,34) austauschbar ist.
